# EUROPÄISCHE PATENTSCHRIFT

(11) **EP 2 920 248 B1**
(45) Veröffentlichungstag und Bekanntmachung des Hinweises auf die Patenterteilung: **07.11.2018**
(21) Anmeldenummer: 13783229.1
(22) Anmeldetag: 18.10.2013
(51) Int. Cl.: C09C 1/02, C04B 22/06, C01F 11/02, C09D 7/62, A61K 8/36, A61Q 11/00, A61Q 19/00, A61K 8/85, C09C 3/00, C09C 3/04, C09C 3/08, C09D 7/40, A61K 8/02, A61K 8/19

(54) **OBERFLÄCHENMODIFIZIERTES KALZIUMOXID UND VERFAHREN ZU DESSEN HERSTELLUNG**
SURFACE-MODIFIED CALCIUM OXIDE AND PROCESS FOR PRODUCING THE SURFACE-MODIFIED CALCIUM OXIDE
OXYDE DE CALCIUM À SURFACE MODIFIÉE ET SON PROCÉDÉ DE FABRICATION

(30) Priorität: 15.11.2012 EP 12007737
(43) Veröffentlichungstag der Anmeldung: 23.09.2015
(73) Patentinhaber: Kalkfabrik Netstal AG, 8754 Netstal (CH)
(72) Erfinder: STARK, Wendelin Jan, CH-8049 Zürich (CH)
(74) Vertreter: Grimm, Siegfried
(86) Internationale Anmeldenummer: PCT/CH2013/000180
(87) Internationale Veröffentlichungsnummer: WO 2014/075197

(56) Entgegenhaltungen:
- WO-A1-94/03536

## Beschreibung

Die vorliegende Erfindung betrifft Verfahren zur Herstellung von oberflächenmodifizierten Kalziumoxidpartikeln umfassend die Herstellungsschritte gemäss Anspruch 12, neuartige Kalziumoxidpartikel gemäss Anspruch 1 sowie deren Verwendungen.

Kalkstein, gebrannter und gelöschter Kalk sind bekannte technische Produkte, Details können Kirk-Othmer (Encyclopedia of Chemical Technology, 2010, Wiley, Stichwort "lime and limestone") entnommen werden. Kalkstein ist in zahlreichen Konsumgütern enthalten, wird in industriellen Prozessen verwendet und dient als Ausgangsmaterial für gebrannten Kalk (ein Gemisch welches überwiegend Kalziumoxid enthält) und gelöschten Kalk (ein Gemisch welches überwiegend Kalziumhydroxid enthält). Gebrannter Kalk wiederum dient als Ausgangsmaterial für gelöschten Kalk. Kalziumoxid ist sehr reaktiv und empfindlich gegenüber Wasser und Feuchtigkeit und hat wegen der allgegenwärtigen Luftfeuchtigkeit nur begrenzte Anwendungsfelder gefunden. Die Empfindlichkeit gegenüber Luftfeuchtigkeit nimmt zu, je kleiner die Kalziumoxid-partikel sind, da das Verhältnis Oberfläche zu Masse sich ungünstig verschiebt.

Nicholson (CH640201) und Brelowski et al (WO2007/092006) beschreiben Verfahren zur Herstellung von gebranntem Kalk mit verbesserter Fliessfähigkeit. Dabei wird vermahlener, gebrannter Kalk in einem handelsüblichen Mischer mit spezifischen Siloxanen vermischt. Die so erhaltenen Kalziumoxidpartikel weisen ein verbessertes Fliessverhalten auf. Für spezifische Anwendungen in der Metallindustrie sind diese Materialien vorteilhaft; für zahlreiche andere Anwendungen sind die Partikel wegen ihrer Siloxan - Modifikation hingegen völlig ungeeignet.

Nakamura et al (WO2005/100246) haben erkannt, dass die Produktion von feinteiligem Kalziumoxid Probleme bereitet (S.2, letzter Satz): "Accordingly, it has been impossible to produce high concentration slurry of calcium oxide nanoparticles by conventional techniques." und schlagen daher einen synthetischen Weg ausgehend von Kalziumdiketonaten vor. Es ist offensichtlich, dass ein derartiger Produktionsweg energieaufwändig, teuer und mit dem Anfall von Nebenprodukten verbunden ist.

Chai et al (WO95/15293) offenbaren ebenfalls ein synthetisches Verfahren zur Herstellung von Kalziumoxidpartikeln durch Zersetzung von Kalziumcarbonat im Plasma. Es ist offensichtlich, dass ein derartiger Produktionsweg energieaufwändig und teuer ist.

Bezze (WO94/03536) offenbart ein Verfahren zur Herstellung von wasserfreiem Kalziumoxid welches mit spezifischen Titanaten oder Zirkonaten beschichtet ist; ferner wird deren Verwendung als Additiv in Polymeren beschrieben. Es ist offensichtlich, dass die eingesetzten Ausgangsmaterialien sehr spezifisch sind und wegen Ihres hohen Preises einer allgemeinen Verwendung entgegenstehen. Ferner wird die Verwendung dieser Übergangsmetalle bei einigen Anwendungen wegen der schlechten biologischen Abbaubarkeit als Nachteil angesehen.

Eine Aufgabe der vorliegenden Erfindung ist daher die Bereitstellung eines verbesserten Verfahrens zur Erzeugung von partikulärem Kalziumoxid mit modifizierter Oberfläche. Von besonderer Bedeutung ist, eine wirtschaftlich operierendes Verfahren und eine entsprechende Anlage bereit zu stellen, die qualitativ hochwertige Produkte erzeugt. Vorteilhaft ist es ferner, den Energieaufwand zu minimieren und natürlich vorkommende Ausgangsmaterialien zu verwenden. Eine weitere Aufgabe der vorliegenden Erfindung ist die Bereitstellung von modifizierten Kalziumoxidpartikeln sowie neuen Anwendungsfeldern für diese Materialien.
Die vorstehend umrissenen Aufgaben werden gemäss den unabhängigen Ansprüchen gelöst. Die abhängigen Ansprüche stellen vorteilhafte Ausführungsformen dar.

Die Erfindung wird nachstehend im Detail erläutert. Sofern sich aus dem direkten Zusammenhang keine andere Bedeutung ergibt, haben die folgenden Begriffe die hier angegebene Bedeutung.

Die im Zusammenhang mit der vorliegenden Erfindung gegebenen allgemeinen, bevorzugten und besonders bevorzugten Ausführungsformen, Bereiche usw. können beliebig miteinander kombiniert werden. Ebenso können einzelne Definitionen, Ausführungsformen usw. entfallen bzw. nicht relevant sein. Der Begriff "umfassend" soll die Bedeutungen "enthaltend" und "bestehend aus" einschliessen.

Prozentangaben sind, soweit nicht anders vermerkt, als Massen-% angegeben.

Der Begriff "gebrannter Kalk" ist bekannt und bspw. in Kirk-Othmer (s.o.) beschrieben und enthält im wesentlichen CaO, typischerweise >90 m-%. In der vorliegenden Erfindung werden daher gebrannter Kalk und Kalziumoxid synonym verwendet. Gemäss der vorliegenden Erfindung liegt Kalziumoxid als partikuläres Material vor. Im Zusammenhang mit der vorliegenden Erfindung werden daher Nanopartikel mit einem Grössenbereich von 10 bis 100 nm definiert; entsprechend Submikron Partikel mit einem Grössenbereich von 100 nm bis 1000 nm, Mikropartikel von 1 bis 40 Mikrometer, bevorzugt von 1 bis 10 Mikrometer Durchmesser. Korngrössen und - verteilung lassen sich mit bekannten Methoden bestimmen. Kleine Partikel (unter 10 Mikrometer Durchmesser) werden insbesondere mittels optischer Detektion (Lichtstreuung), durch Mikroskopie (Licht-Mikroskopie oder Elektronenmikroskopie; Ausmessen der einzelnen Partikel und Berechnung der mittleren Partikelgrösse), oder in optischen Zentrifugen (z. B. Lumisizer, LUM GmbH oder in einer Röntgenzentrifuge, z.B. BI-XDC von "Brookhaven Instruments Corporation") die direkt einen hydrodynamischen Durchmesser liefern, gemessen.

Der Begriff "Partikel- oder Korngrösse" wird synonym verwendet, ist allgemein bekannt und kann nach gängigen Methoden bestimmt werden. Sofern sich in der vorliegenden Erfindung nichts anderes ergibt, wird die Partikelgrösse als "mittlere Partikelgrösse" angegeben. Für Partikel im Mikrometer Bereich (Durchmesser über 10 Mikrometer) erfolgt die Messung bevorzugt mittels Lasergranulometrie (z.B. Sympatec HELOS). Für Partikel über 100 Mikrometer Grösse wird bevorzugt eine Siebanalyse angewendet. Eine Angabe 0-200µm als Resultat einer Siebanalyse ist dahin zu verstehen, dass der obere Grenzwert für die Partikelgrösse 200µm beträgt (bspw. durch Bestimmung des Siebrückstandes durch Luftstrahlsiebung), der untere Grenzwert jedoch in einem sinnvollen technischen Bereich liegt (bspw. wie durch ein Mahlverfahren gegeben).

Nachfolgend wird die Erfindung, insbesondere Verfahren und Materialien, unter Bezugnahme auf die **Figuren** näher erläutert. Es stehen (M) für Mühle / Mahlvorrichtung, (S) für Sprühvorrichtung, (T) für Trennvorrichtung, (W) für Wärmetauscher sowie r-CaO und m-CaO für rohen bzw. modifizierten gebrannten Kalk, MH für Mahlhilfsmittel und Add für Additiv.

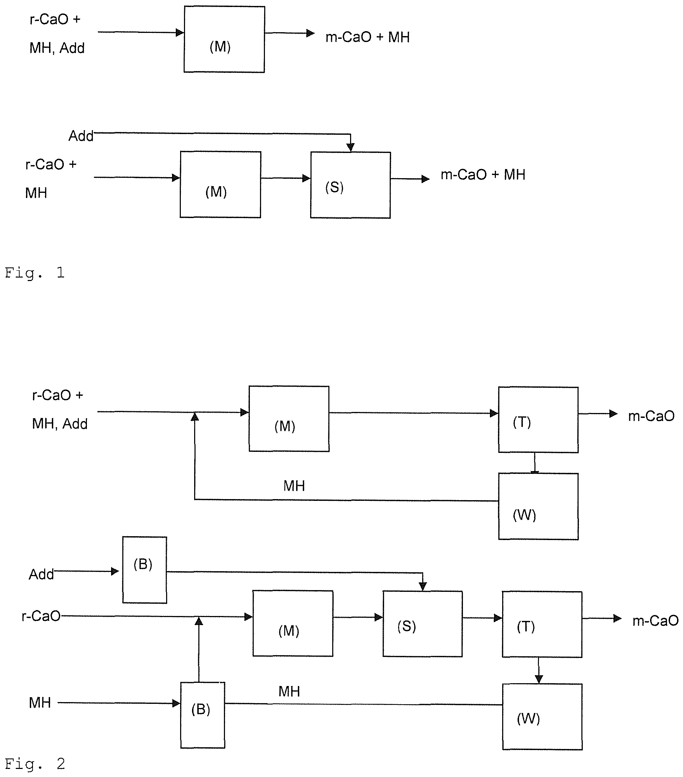

Fig. 1 zeigt schematisch das erfindungsgemässe Verfahren zur Herstellung von gebranntem Kalk, wobei in der oberen Grafik die Ausgestaltung nach Methode (I) mit gleichzeitigem Mahlen und Modifizieren mittels Additiv und in der unteren Grafik die Ausgestaltung nach Methode (II) mit aufeinanderfolgenden Schritten für Mahlen und Modifizieren mittels Additiv dargestellt sind.

Fig. 2 zeigt schematisch das erfindungsgemässe Verfahren zur Herstellung von gebranntem Kalk mit zusätzlichem Trenn- und Rezyklierungsschritt des Mahlhilfsmittels MH. Dabei wird das zunächst erhaltene Gemisch aus modifiziertem gebrannten Kalk m-CaO und Mahlhilfsmittel MH in einer Trennvorrichtung (T) getrennt, das Mahlhilfsmittel in einem Wärmetauscher gekühlt und in den Prozess rezykliert. In der oberen Grafik wird dabei die Ausgestaltung nach Methode (I) und in der unteren Grafik die Ausgestaltung nach Methode (II) dargestellt. In der unteren Grafik wird zusätzlich die Verwendung von Zwischenlagern (B) für Additiv und Mahlhilfsmittel dargestellt.

In einem **ersten Aspekt** bezieht sich die Erfindung daher auf ein Verfahren zur Herstellung von oberflächenmodifiziertem Kalziumoxidpartikeln , wie in Anspruch 1 definiert, aus rohem Kalziumoxid, Additiv(en) und Mahlhilfsmittel(n) in einer inerten, gegen die Umgebung abgedichteten Mahlvorrichtung.

In einer ersten Ausgestaltung (Methode I) betrifft die Erfindung ein Verfahren zur Herstellung von oberflächenmodifiziertem Kalziumoxidpartikeln, umfassend die Schritte (a) Bereitstellen von rohem Kalziumoxid, Additiv, Mahlhilfsmittel, (b) Bereitstellen und Inertisieren einer gegen die Umgebung abgedichteten Mahlvorrichtung (M), (c) Mahlen des rohen Kalziumoxids in Gegenwart von Mahlhilfsmittel und Additiv in dieser Mahlvorrichtung.

In einer zweiten Ausgestaltung (Methode II) betrifft die Erfindung ein Verfahren zur Herstellung von oberflächenmodifizierten Kalziumoxidpartikeln, umfassend die Schritte (a) Bereitstellen von rohem Kalziumoxid, Additiv, Mahlhilfsmittel; (b) Bereitstellen und Inertisieren von gegen die Umgebung abgedichteten Mahlvorrichtung (M) und Sprühvorrichtung (S); (c) Mahlen des Rohen Kalziumoxids in Gegenwart von Mahlhilfsmittel in besagter Mahlvorrichtung (M); (d) Kontaktieren des gemahlenen Kalziumoxids mit Additiv in besagter Sprühvorrichtung (S).

Die Methoden (I) und (II) unterscheiden sich demnach im Wesentlichen darin, ob das Vermahlen des rohen Kalziumoxids und das Kontaktieren mit Additiv simultan (Methode I) oder in aufeinanderfolgenden Schritten (Methode II) erfolgt. In beiden Methoden können weitere Schritte vor- und / oder nachgeschaltet sein. Dieser Aspekt der Erfindung, einschliesslich einer Beschreibung der einzelnen Prozessschritte und verwendeten Apparate und Ausgangsmaterialien, soll nachstehend erläutert werden. **Ausgangsmaterialien:** Die Ausgangsmaterialien, rohes Kalziumoxid (r-CaO), Additiv(e) (Add), Mahlhilfsmittel (MH) sind an sich bekannt und kommerziell erhältlich oder nach allgemein bekannten Methoden herstellbar. Erfindungsgemäss können diese Materialien als reine Komponente, als Gemisch verschiedener Komponenten und in unterschiedlicher Reinheit vorliegen. So ist bspw. ein Bezug auf "Additiv" als "ein Additiv" oder "ein Additivgemisch" zu verstehen.

**Rohes Kalziumoxid:** Rohes Kalziumoxid ist ein grosstechnisches Produkt und in unterschiedlichen Reinheitsgraden unter der Bezeichnung "gebrannter Kalk" verfügbar. Das erfindungsgemässe Verfahren ist vom Reinheitsgrad unabhängig. Besonders geeignet ist das vorliegende Verfahren, wenn das rohe Kalziumoxid gebrannter Kalk ist, welcher aus natürlichem Kalkstein durch Brennen bei ca. 900 - 1100°C gewonnen wird. Solch natürlicher Kalkstein kann kalziumreicher Kalkstein, magnesiumreicher Kalkstein oder Dolomit sein. Bevorzugt wird kalkreicher Kalkstein mit tiefem Schwermetallgehalt. Der Begriff "roh" ist im Zusammenhang mit der vorliegenden Erfindung auf die chemische Zusammensetzung bezogen. Der Begriff rohes Kalziumoxid schliesst daher sowohl Kalziumoxid mit einer Grösse 0- 130 mm ein ("Stückkalk") als auch mit einer Grösse 0-200 µm ("Feinkalk").

**Additiv:** Das Additiv führt zu einer oberflächlichen Modifikation des vermahlenen Kalziumoxids. Für das erfindungsgemässe Verfahren sind nichtwässrige Flüssigkeiten (besonders im Falle von Nassmühlen (M)) geeignet. Die Art der Modifikation hängt von der Wahl des Additivs und den Verfahrensbedingungen ab, grundsätzlich kann das Additiv durch chemische Bindung (insbesondere ionische Bindung) oder Sorption (insbesondere Adsorption) erfolgen.

In einer Ausführungsform ist das Additiv daher eine Flüssigkeit aus der Gruppe der C₄₋₂₀-Phosphonsäuren. Erfindungsgemäss können solche Flüssigkeiten entweder (i) aus diesen Verbindungen bestehen oder (ii) diese Verbindungen und zusätzlich nichtwässriges Verdünnungsmittel enthalten. Die Zugabe eines Verdünnungsmittels ist insbesondere dann vorteilhaft, wenn die genannten Verbindungen bei Herstellungsbedingungen nicht flüssig vorliegen.

In einer weiteren Ausführungsform ist das Additiv eine Flüssigkeit aus der Gruppe Ölsäure und Stearinsäure. Die Verwendung dieser natürlichen Fettsäuren ist aus verschiedenen Gründen vorteilhaft. Zum einen ist das erhaltene Produkt biologischen Ursprungs, biologisch abbaubar und toxikologisch unbedenklich. Zum anderen ist die Produktion unproblematisch, da weder Emission von VOC noch Explosions-gefährdung zu erwarten sind. Erfindungsgemäss können solche Flüssigkeiten entweder (i) aus diesen Verbindungen bestehen oder (ii) diese Verbindungen und zusätzlich nichtwässriges Verdünnungsmittel enthalten. Die Zugabe eines Verdünnungsmittels ist insbesondere dann vorteilhaft, wenn die genannten Verbindungen bei Herstellungsbedingungen nicht flüssig vorliegen

**Mahlhilfsmittel:** Als Mahlhilfsmittel werden die Komponenten verstanden, welche eine Zerkleinerung des Ausgangsmaterials bewirken. Für das erfindungsgemässe Verfahren sind Gase (im Falle eine Mahlvorrichtung M aus der Gruppe der Gasstrahlmühlen) oder nichtwässrige Flüssigkeiten (Im Falle eine Mahlvorrichtung M aus der Gruppe der Nassmühlen) geeignet. Es hat sich als wichtig herausgestellt, dass der Wassergehalt des Mahlhilfsmittels niedrig ist. Ein niedriger Wassergehalt kann durch entsprechend spezifizierte Ausgangsmaterialien sichergestellt werden oder durch Rezyklieren des Mahlhilfsmittels. Es wird davon ausgegangen, dass das rohe Kalziumoxid während des Mahlvorgangs eine wasserentziehende Wirkung zeigt.

In einer Ausführungsform ist das Mahlhilfsmittel daher ein inertes Gas mit einem Wassergehalt < 0.1 g Wasserdampf pro Kubikmeter, bevorzugt < 0.01 g Wasserdampf pro Kubikmeter. Dies kann durch Einsatz von Gasen mit entsprechender Spezifikation sichergestellt werden. Solche Gase sind kommerziell erhältlich. Alternativ kann das Gas wie nachstehend beschrieben rezykliert werden. Bevorzugte Gase sind Stickstoff, Edelgase, getrocknete Luft.

In einer weiteren Ausführungsform ist das Mahlhilfsmittel eine nichtwässrige Flüssigkeit mit einem Wassergehalt < 0.1 g Wasser pro Liter, bevorzugt < 0.05 g Wasser pro Liter, am besten < 0.02 g Wasser pro Liter. Dies kann durch Einsatz von Flüssigkeiten mit entsprechender Spezifikation sichergestellt werden. Solche Flüssigkeiten sind kommerziell erhältlich. Alternativ kann die Flüssigkeit wie nachstehend beschrieben rezykliert werden. Bevorzugte Flüssigkeiten sind pflanzliche Oele, Derivate von pflanzlichen Ölen, Paraffine, Glykole und Glykolether.

Je nach Ausgestaltung der Mahlvorrichtung kann zusätzlich ein festes Mahlhilfsmittel (bspw. Stahl- oder Keramik-kugeln) eingesetzt werden. Dies erhöht die Effizienz des Schrittes (c), erfordert aber einen zusätzlichen Trennschritt (e).
**Schritt (a):** Die Bereitstellung der Ausgangsmaterialien erfolgt in an sich bekannter weise. Dies kann kontinuierlich oder diskontinuierlich erfolgen. Zweckmässig werden die Ausgangsmaterialien aus Zwischenlagern (Behälter (B)) zur Verfügung gestellt, diese sind bevorzugt gegen Feuchtigkeit aus der Umgebung abgeschirmt. Das rohe Kalziumoxid kann direkt von einer Mahlanlage zugeführt werden; typischerweise mit einer Partikelgrösse 0-200µm.
**Schritt (b):** Es hat sich als wichtig für das erfindungsgemässe Verfahren herausgestellt, dass der Mahlvorgang in Abwesenheit von Wasser durchgeführt wird. Entsprechend wird unter Inertisieren im Zusammenhang mit der vorliegenden Erfindung die Entfernung von Wasser verstanden. Dies schliesst auch die Entfernung von adsorbiertem Wasser an Oberflächen der Mahlvorrichtung ein.

In einer Ausführungsform erfolgt das Inertisieren (Trocknen) indem die Mahlvorrichtung (M) und ggf. Sprühvorrichtung (S) erhitzt werden, ggf. unter reduziertem Druck. Geeignet ist bspw. eine Erwärmung auf 120°C, 1h. Diese Ausführungsform ist besonders geeignet für kleinere Mahlvorrichtungen und / oder diskontinuierliche Verfahren.

In einer alternativen Ausführungsform erfolgt die Inertisierung durch das eingesetzte rohe Kalziumoxid. In dieser Ausführungsform wird Schritt (c) durchgeführt, bis ein Produkt gleichbleibender Qualität erzeugt wird. Diese Ausführungsform ist besonders geeignet für grössere Mahlvorrichtungen bei denen eine Erwärmung nicht möglich ist und / oder bei kontinuierlichen Verfahren. In dieser Ausführungsform wird das hier beschriebene Verfahren durchgeführt, wobei sich anfänglich ein Produkt mit verminderter Qualität bildet, insbesondere mit einem erhöhten Anteil an Kalziumhydroxid und / oder unzureichender Oberflächenmodifikation. Es wird davon ausgegangen, dass während des Mahlvorgangs vorhandenes Wasser sich nachteilig auf das Verfahren auswirkt, dieses Wasser jedoch durch das eingesetzte rohe Kalziumoxid gebunden wird. Das zunächst entstehende Produkt wird in dieser Ausführungsform entsorgt beziehungsweise anderweitig verwendet.

In einer weiteren alternativen Ausführungsform erfolgt die Inertisierung durch das eingesetzte rohe Kalziumoxid mit prozessinterner Rezyklierung. In dieser Ausführungsform wird Schritt (c) durchgeführt, bis ein Produkt gleichbleibender Qualität erzeugt wird und das vorgängig produzierte Produkt geringer Qualität für die Bereitstellung von rohem Kalziumoxid genutzt wird (Rezyklierung). So ist es möglich, dieses zunächst produzierte Produkt einem Kalkbrennofen - ggf. nach weiteren Bearbeitungsschritten - zuzuführen um rohes Kalziumoxid zu erhalten. In einer alternativen Ausführungsform wird das teilweise hydratisierte Kalziumoxid extern verwertet (z.B. in der Boden-Stabilisierung).
**Schritt (c):** Für das Vermahlen sind grundsätzlich alle Mahlvorrichtungen (Mühlen (M)) geeignet, die bisher zum Mahlen von Kalziumoxid verwendet werden. Insbesondere sind Gasstrahlmühlen (Jet Mill) und Nassmühlen (Wet mill) geeignet. Die Auswahl und Auslegung entsprechender Vorrichtungen ist dem Fachmann geläufig.

Um zu gewährleisten, dass das Vermahlen in Abwesenheit von Wasser erfolgt, muss die Mahlvorrichtung gegen die Umgebung abgedichtet sein. So wird gewährleistet, dass kein Wasser (insbesondere durch Luftfeuchtigkeit) in den Prozess gelangt. Eine entsprechende Abdichtung kann durch die Konstruktion der Mühle gewährleistet werden und ist dem Fachmann geläufig. Ferner kann es vorteilhaft sein, diesen Schritt unter leichtem Überdruck, bspw. 0.1 bar, durchzuführen.

In einer Ausführungsform werden die Ausgangsmaterialien Kalziumoxid, Additiv und Mahlhilfsmittel gleichzeitig der Mühle zugeführt. Somit erfolgen die Zerkleinerung und die Oberflächenmodifikation simultan.

In einer weiteren Ausführungsform werden die Ausgangsmaterialien Kalziumoxid und Mahlhilfsmittel gleichzeitig der Mühle zugeführt, das Additiv in einer nachgeschalteten Sprühvorrichtung (S). Somit erfolgen die Zerkleinerung und die Oberflächenmodifikation in aufeinanderfolgenden Teilschritten.

In einer weiteren Ausführungsform werden die Ausgangsmaterialien Kalziumoxid, Additiv und Mahlhilfsmittel gleichzeitig der Mühle zugeführt und zusätzlich wird Additiv in einer nachgeschalteten Sprühvorrichtung (S) aufgebracht. Somit erfolgen die Zerkleinerung und die Oberflächenmodifikation sowohl simultan als auch konsekutiv. Diese Ausführungsform ermöglicht es in einfacher Weise, eine Oberflächenmodifikation mit verschiedenen Additiven zu realisieren.
**Schritt (d):** Für das Kontaktieren des gemahlenen Kalziumoxids mit Additiv(en) sind grundsätzlich bekannte Vorrichtungen (wie Sprüher, Vernebler, Trommelwäscher und dgl.) geeignet, die bekannterweise zum Beschichten von Partikeln geeignet sind. Insbesondere geeignet sind kontinuierliche Mischsysteme (wie Vrieco-Nauta Continuous Mixer; bspw. Hosokawa Micron LTD) ausgestattet mit Zerstäuberdüsen (zwecks Feinstverteilung der Additive, bspw. Firma Lechler). Die

Auswahl und Auslegung entsprechender Vorrichtungen ist dem Fachmann geläufig.

In einer Ausgestaltung wird das Additiv zunächst mit einem Verdünnungsmittel verdünnt und dann mit dem gemahlenen Kalziumoxid kontaktiert. Das Verdünnungsmittel kann anschliessend vom erhaltenen Produkt abgetrennt werden. Geeignete Verdünnungsmittel sind bspw. wasserfreie Alkohole (wie Ethanol), wasserfreie kurzkettige Kohlenwasserstoffe (wie Paraffinöl) und wasserfreie Halogenkohlenwasserstoffe (wie Methylenchlorid). Diese Ausgestaltung ist besonders für den kleineren Produktionsmassstab, wie Forschungs- und Technikumsanlagen, vorteilhaft.
**Schritt (e):** Das aus dem Schritt (c) bzw. (d) erhaltene Produkt ist zunächst ein Gemisch enthaltend oberflächenmodifiziertes Kalziumoxid, Mahlhilfsmittel und ggf. Additiv. In einer Ausführungsform kann dieses in einem nächsten Schritt in einer Trennvorrichtung (T) aufgetrennt werden. Geeignete Trennverfahren und Vorrichtungen sind an sich bekannt. Sofern das Mahlhilfsmittel eine Flüssigkeit ist, sind alle Vorrichtungen zum Trennen von Flüssig-Fest Gemischen, bspw. Zentrifugen und Filter, geeignet. Sofern das Mahlhilfsmittel ein Gas ist, sind alle Vorrichtungen zum Trennen von Gas-Fest-Gemischen, bspw. Fliehkraftabscheider, geeignet.

Wie ausgeführt, ist Schritt (e) fakultativ; ein eigener Trennschritt ist nicht zwingend nötig. Im Falle gasförmiger Mahlhilfsmittel können diese am Ende des Mahlvorgangs (c) bzw. Sprühvorgangs (d) einfach in die Umgebung entweichen. Dies ist besonders dann vorteilhaft, wenn keine Rückführung des Mahlhilfsmittels vorgesehen ist. Ferner kann sich eine Auftrennung erübrigen, wenn das Mahlhilfsmittel eine Suspension ist, die in der weiteren Produktanwendung verwendet werden soll. In diesem Fall betrifft die Erfindung in einer weiteren Ausführungsform ein Verfahren zur Herstellung einer Suspension, enthaltend eine nichtwässrige Flüssigkeit (kontinuierliche Phase) und oberflächenmodifizierte Kalziumoxidpartikel (disperse Phase).
**Schritt (f):** Sofern in Schritt (e) Mahlhilfsmittel und / oder Additive vom Produkt abgetrennt werden, können diese in das Verfahren rezykliert werden. Gegebenenfalls ist es vorteilhaft, bei der Rezyklierung eine Kühlung mittels Wärmetauscher (W) vorzusehen.

**Allgemeine Verfahrensmerkmale:** Nachfolgend werden einige allgemeine und bevorzugte Ausgestaltungen des erfindungsgemässen Verfahrens dargelegt.

Das hier beschriebene Verfahren kann kontinuierlich, teilkontinuierlich oder diskontinuierlich ausgestaltet sein. Bei einem kontinuierlichen Verfahren erfolgt zumindest Zufuhr zur und Entnahme aus der Mahlvorrichtung (M) kontinuierlich. Bei einem diskontinuierlichen Verfahren erfolgt Zufuhr zur und Entnahme aus der Mahlvorrichtung (M) absatzweise.

Die Partikelgrösse der erfindungsgemäss hergestellten oberflächenmodifizierten Kalziumoxidpartikel kann einen weiten Bereich annehmen und ist im Wesentlichen von der Mahlvorrichtung abhängig. Typische Werte für einen mittleren Durchmesser sind < 30 µm, bevorzugt < 10 µm, besonders bevorzugt < 2 µm. Die Partikelgrösse kann an die angestrebte Verwendung angepasst werden.

Der Gehalt an Additiv der erfindungsgemäss hergestellten oberflächenmodifizierten Kalziumoxidpartikel kann einen weiten Bereich annehmen und hängt ab von der Einsatzmenge, der Partikelgrösse und den Verfahrensparametern. Typische Werte sind < 20 m-%, bevorzugt < 8 m-%, besonders bevorzugt < 2 m-% ist, bezogen auf die Masse an Kalziumoxid.

Die Partikelgrösse des eingesetzten rohen Kalziumoxids kann einen weiten Bereich annehmen und ist im Wesentlichen von der Mahlvorrichtung abhängig. Typische Werte für einen mittleren Durchmesser sind in Partikeln mit einer Grösse 40 Mikrometer bis 2 Millimeter. Es hat sich ferner als günstig erwiesen, die Partikelgrösse in der Mahlvorrichtung um den Faktor 10 bevorzugt um den Faktor 20 zu reduzieren.

Die Reaktionsfähigkeit des eingesetzten rohen Kalziumoxids kann einen weiten Bereich annehmen und ist im Wesentlichen durch dessen Herstellung bestimmt. Die Reaktionsfähigkeit von Kalziumoxid wird nach EN 459-2 bestimmt (Baukalk - Teil 2: Prüfverfahren; Nasslöschkurve) . Hier hat sich der t₆₀-Wert als messbare Eigenschaft von Kalziumoxid durchgesetzt. Für das vorliegende Verfahren sind t₆₀-Werte unter 5 Minuten geeignet; unter 2 Minuten bevorzugt; besonders geeignet sind t₆₀-Werte von 15 - 60 s.

Wie ausgeführt, können die verschiedenen Merkmale des erfindungsgemässen Verfahrens beliebig miteinander kombiniert werden. Es haben sich jedoch bestimmte Kombinationen von Merkmalen als vorteilhaft erwiesen, diese sind besonders bevorzugt und in nachfolgender Tabelle zusammengefasst:

| Ausführungsform | A | B | C | D |
|---|---|---|---|---|
| Methode | I | I | II | II |
| Mahlhilfsmittel | Gasförmig | Gasförmig | flüssig | flüssig |
| Additiv | flüchtige Metallverb. | flüchtige Metallverb. | Carbons. Derivat | Phosphonsäuren |
| Typ Mahlvorr. | Jet Mill | Jet Mill | Wet Mill | Wet Mill |
| Typ Sprühvorr. | 2 Phasen-Düse | Düse | Düse | keine |
| Typ Trennvorr. | nein | Zyklon | Zentrifuge | Hydrozyklon |
| Rezyklieren Mahlhilfsmitel | nein | ja | ja | ja |

In einem **zweiten Aspekt** beschreibt diese Erfindung oberflächenmodifizierte Kalziumoxidpartikel erhältlich nach oder erhalten nach einem Verfahren wie vorstehend beschrieben. Dieser Aspekt der Erfindung soll nachstehend näher erläutert werden. Diese neuartigen Materialien sind auch Gegenstand der vorliegenden Erfindung. Somit beschreibt dieses Dokument oberflächen-modifizierte Kalziumoxidpartikel erhältlich nach einem Verfahren wie hier beschrieben und oberflächenmodifizierte Kalziumoxidpartikel erhalten nach einem Verfahren wie hier beschrieben.

In einer vorteilhaften Ausführungsform betrifft die Erfindung oberflächenmodifizierte Kalziumoxidpartikel mit
▪ einer Partikelgrösse von 1 bis 10 Mikrometer,
▪ einem Gehalt an Oelsäure von 0.5-5%, bevorzugt 0.5-4 %,
▪ einem Gehalt an Kalziumoxid von 95 bis 99 %.

In einer vorteilhaften Ausführungsform betrifft die Erfindung oberflächenmodifizierte Kalziumoxidpartikel mit
▪ einer Partikelgrösse von 1 bis 10 Mikrometer,
▪ einem Gehalt an Stearinsäure von 0.5 bis 5 %,
▪ einem Gehalt an Kalziumoxid von 95 bis 99.5 %.

In einer vorteilhaften Ausführungsform betrifft die Erfindung oberflächenmodifizierte Kalziumoxidpartikel mit
▪ einer Partikelgrösse von 1 bis 10 Mikrometer,
▪ einem Gehalt an Dodecyl-Phosphonsäure von 0.25 bis 2 %,
▪ einem Gehalt an Kalziumoxid von 96 bis 99 %.

In einer weiteren Ausführungsform betrifft die Erfindung ein Gemisch aus Kalziumoxid und flüssigem Mahlhilfsmittel. In dieser Ausführungsform wird das primär erzeugte Produkt nicht getrennt. Ein solches Produktgemisch kann als Dispersion von oberflächenmodifiziertem Kalziumoxid verwendet werden. In dieser Ausführungsform betrifft die Erfindung eine Dispersion, wobei
▪ die disperse Phase Kalziumoxid mit einer Partikelgrösse von 0.5 bis 2 Mikrometer, einem Gehalt an Ölsäure oder Stearinsäure von 0.2 bis 5 %, einem Gehalt an Kalziumoxid in den Partikeln von 90 bis 99 % bildet und
▪ die kontinuierliche Phase Rapsöl enthält oder daraus besteht und
▪ die Dispersion einem Gehalt an Partikel von 5 bis 20 % aufweist.

In einem **dritten Aspekt** bezieht sich die Erfindung auf die Verwendung der hier beschriebenen Kalziumoxidpartikel sowie Produkte geeignet für diese Verwendungen. Dieser Aspekt der Erfindung soll nachstehend näher erläutert werden.

Die Kalziumoxidpartikel erhältlich oder erhalten nach dem hier beschriebenen Verfahren können in den bisher bekannten Verwendungen für Kalziumoxid verwendet werden. In diesen Verwendungen zeigt das erfindungsgemässe Kalziumoxid aufgrund seiner verbesserten Eigenschaften Vorteile gegenüber konventionellem Kalziumoxid. So führt die reduzierte Reaktivität gegenüber Umgebungsluft zu verbesserter Handhabbarkeit und Lagerfähigkeit.

Zusätzlich ergeben sich für die hier beschriebenen Kalziumoxidpartikel weitere Anwendungen. So wird bei den erfindungsgemässen Kalziumoxidpartikeln eine verbesserte Benetzbarkeit und / oder Mischbarkeit beobachtet, was neue Anwendungsfelder ermöglicht. Die Erfindung betrifft daher auch die Verwendung von oberflächenmodifizierten Kalziumoxidpartikeln
▪ als Pflanzenbehandlungsmittel;
▪ als Füllstoff, insbesondere als funktionaler Füllstoff, in Kunststoffen;
▪ als Füllstoff (und ggf. Pigment) in Lacken oder Farben;
▪ als Lebensmitteladditiv;
▪ als Additiv in Kosmetika;
▪ als Hilfsstoff in pharmazeutischen Produkten;
▪ als Additiv in Papiererzeugnissen;
▪ als Haftvermittler in Leim- und Polymerformulierungen;
▪ als Hilfsstoff in Zement- oder Betonmischungen.

Die hier beschriebenen Kalziumoxidpartikel können handelsüblichen Produkten beigemischt werden, um in den vorstehend genannten Verwendungen eingesetzt zu werden.

In einer Ausführungsform betrifft die Erfindung daher ein **Pflanzenbehandlungsmittel**, insbesondere aus der Gruppe der Fungizide und Bakterizide, enthaltend Kalziumoxidpartikel wie hier beschrieben. Ein bevorzugtes Anwendungsgebiet des erfindungsgemässen CaO ist als lokal wirkendes Mittel gegen Pflanzenkrankheiten, insbesondere falschem Mehltau, Apfelschorf und/oder Feuerbrand. Die Kalziumoxidpartikel können darin in einem weiten Konzentrationsbereich vorliegen, typischerweise in einer Menge von 1 bis 20 % (insbesonder im Falle flüssiger Formulierungen) oder in einer Menge von 75-100% (insbesondere im Falle fester Formulierungen).

Dabei hat das hier beschriebene modifizierte CaO eine Reihe von Vorteilen: Zunächst zeigt das Produkt eine hohe Umweltverträglichkeit und keine Wassergefährdung, da CaO in der Umwelt nur eine begrenzte Lebenszeit hat, und innert Stunden bis Tagen zu Kalkstein (Calciumcarbonat) reagiert, somit also in ein natürliches und bekanntes Produkt übergeht. Ferner ist das Basismaterial CaO und das Schichtmaterial (Additiv) zu günstigen Preisen erhältlich, so dass das Produkt auch wirtschaftlich interessant ist. Besonders hervorzuheben ist jedoch seine gute Wirksamkeit: In Vergleichsversuchen (siehe nachfolgende Beispiele 4.1, 4.2 und 4.3) wurde sowohl das erfinderische, modifizierte CaO wie auch nicht-modifiziertes (reines) CaO sowie Polymer-beschichtetes CaO in Wirksamkeitsversuchen gegen Pflanzenschädlinge untersucht. Sowohl die Versuchreihen in Beispielen 4.1, 4.2, wie auch in 4.3, zeigen, dass das erfinderische CaO eine verbesserte Wirksamkeit gegen die Mikroorganismen hat. Dies ist in 4 vergleichenden Beispielen sowohl für den Anteil an befallenen Blättern (Einträge links / Mitte in den Tabellen), sowie den Schweregrad des Befalls (Anteil toter Blätter, rechts in den Tabellen) gültig.

Die vorliegende Erfindung betrifft daher auch die Verwendung der hier beschriebenen Kalziumoxidpartikel als Pflanzenbehandlungsmittel im Obst-, Gemüse- und Weinbau. Die vorliegende Erfindung betrifft ferner Formulierungen für den Pflanzenschutz enthaltend die hier beschriebenen Kalziumoxidpartikel, insbesondere zur Behandlung von Obstkulturen, Gemüsekulturen und / oder Weinbaukulturen.

In einer weiteren Ausführungsform betrifft die Erfindung einen **Kunststoff** enthaltend Kalziumoxidpartikel wie hier beschrieben, insbesondere aus der Gruppe der Polyolefine, der Polyether, Polysulfone, Polyamide und Polyester. Diese können auch halogeniert sein, insbesondere PVC. Die Kalziumoxidpartikel können darin in einem weiten Konzentrationsbereich vorliegen, typischerweise in einer Menge von 0.2 bis 40%.

In einer weiteren Ausführungsform betrifft die Erfindung **Lacke oder Farben** enthaltend Kalziumoxidpartikel wie hier beschrieben, insbesondere aus der Gruppe umfassend Polyacrylate, Polyester und Polyurethane. Die Kalziumoxidpartikel können darin in einem weiten Konzentrationsbereich vorliegen, typischerweise in einer Menge von 0.2 bis 40%.

In einer weiteren Ausführungsform betrifft die Erfindung **Lebensmittel** enthaltend Kalziumoxidpartikel wie hier beschrieben, insbesondere aus der Gruppe umfassend Back- und Süsswaren, aber auch Fisch, Fleisch und Wein. Die Kalziumoxidpartikel können darin in einem weiten Konzentrationsbereich vorliegen, typischerweise in einer Menge von 0.05 bis 2%.

In einer weiteren Ausführungsform betrifft die Erfindung **kosmetische Artikel** enthaltend Kalziumoxidpartikel wie hier beschrieben, insbesondere aus der Gruppe der Hautpflegeprodukte und Zahnpflegeprodukte. Die Kalziumoxidpartikel können darin in einem weiten Konzentrationsbereich vorliegen, typischerweise in einer Menge von 0.5 bis 20%, insbesondere 0.5 bis 10%.

In einer weiteren Ausführungsform betrifft die Erfindung **pharmazeutische Formulierungen** enthaltend Kalziumoxidpartikel wie hier beschrieben, insbesondere aus der Gruppe der festen Formulierungen, bspw. Tabletten, Pulver, Suppositorien. Die Kalziumoxidpartikel können darin in einem weiten Konzentrationsbereich vorliegen, typischerweise in einer Menge von 0.05 bis 10%.

In einer weiteren Ausführungsform betrifft die Erfindung ein **Papiererzeugnis** enthaltend Kalziumoxidpartikel wie hier beschrieben, insbesondere Papier für Tintenstrahldrucker. Die Kalziumoxidpartikel können darin in einer oberen Schicht (insbesondere der obersten oder der zweitobersten Schicht) vorliegen, welche die Tinte aufnimmt.

In einer weiteren Ausführungsform betrifft die Erfindung eine **Leim- bzw**. **Polymerformulierung** enthaltend Kalziumoxidpartikel wie hier beschrieben, insbesondere zur Verwendung von Dichtungsmaterialien, Fugenabdichtungen, Bauabdichtungen und/oder Polymer-/Klebeanwendungen. Diese Leim bzw. Polymerformulierungen bewähren sich insbesondere auf nassen Oberflächen. Solche Formulierungen sind typischerweise halbflüssig oder hochviskos; die Kalziumoxidpartikel können darin suspendiert vorliegen; geeignete Mengen liegen im Bereich von 0.5 bis 40%, insbesondere 1 bis 40%.

In einer weiteren Ausführungsform betrifft die Erfindung **zementöse Mischungen** sowie **Beton**. enthaltend Kalziumoxidpartikel wie hier beschrieben Die Kalziumoxidpartikel können darin in einem weiten Konzentrationsbereich vorliegen; eine effektive Menge kann der Fachmann anhand von Reihenversuchen ermitteln. Somit betrifft die Erfindung auch die Verwendung der erfindungsgemässen Kalziumoxidpartikel im Zement herstellenden und Zement verarbeitenden Gewerbe ("Zementindustrie").

Die nachstehend genannten **Beispiele** dienen der weiteren Erläuterung der Erfindung; sie sollen die Erfindung in keiner Weise limitieren.

### Bsp.1

In diesem Beispiel wird gezeigt, dass ein Vermahlen von rohem gebranntem Kalk möglich ist, ohne die Reinheit negativ zu beeinflussen. 2 g des Ausgangsmaterials (Partikelgrösse <200µm) werden mit 20 ml Rapsöl und 10 Gramm Mahlkugeln (Zirkon, 0.2 mm Durchmesser) in einer Mühle vom Typ Planetenmühle (Firma Fritsch, Pulverisette 7) für 20 Minuten bei 1100 Umdrehungen pro Minute) vermahlen.

**Quantitative Zunahme von Verunreinigungen in behandeltem rohem Kalk.**

| Nr | Oberflächenbehandlung | prozessbedingtes Kalziumhydroxid /mass-% ^{b} | prozessbedingtes Kalziumkarbonat /mass-% ^{b} |
|---|---|---|---|
| 1 | keine (nur wet milling gemäss hier spezifizierter Bedingungen) | < 1 mass-% | < 1 mass-% |
| 2 | Soy lecithine (2.5 mass-% ^{a}) | < 1 mass-% | < 1 mass-% |
| 3 | Oelsäure (0.25 mass-% ^{a}) | < 1 mass-% | < 1 mass-% |
| 4 | Oelsäure (2.5 mass-% ^{a}) | < 1 mass-% | < 1 mass-% |
| 5 | Stearinsäure (2.5 mass-% ^{a}) | < 1 mass-% | < 1 mass-% |
| 6 | Tetradecylphosphonic acid (0.25 mass-% ^{a}) | < 1 mass-% | < 1 mass-% |

| | | | |
|---|---|---|---|
| ^{a} Oberflächenbehandlungsmittel: Massen-% in Bezug auf Gesamtmasse Feststoffe ^{b} Verunreinigungen eingeführt durch Mahlen (milling (Eintrag 1) oder Mahlen und Oberflächenmodifikation (Einträge 2-6)). | | | |

Die Tabelle zeigt die Zunahme an Kalziumhydroxid und Kalziumkarbonat durch das erfindungsgemässe Verfahren. Diese Werte sind sehr niedrig und gelten für Ausgangsmaterialien hoher Qualität (jeweils 0.5 m-% Kalziumhydroxid und -karbonat) und geringer Qualität (3 m-% Kalziumhydroxid und 5 m-% -karbonat).

### Bsp. 2

In diesem Beispiel wird gezeigt, dass rohes Kalziumoxid aus einer Kalkmahlanlage eingesetzt werden kann und Partikel verschiedener Grösse zu erhalten. Die Partikelgrössen wurden als hydrodynamische Partikelgrössen-verteilung angegeben und mittels Lumisizer bestimmt. Als Mahlhilfsmittel werden zusätzlich 0.2-mm-Kugeln eingesetzt.

**Partikelgrössenverteilung.**

| Nr | Mahlhilfsmittel, | Zeit / min | Additiv | d10 / µm | d50 / µm | d90 / µm |
|---|---|---|---|---|---|---|
| 7 | 0.2 mm; Diethyleneglycolmonobutyl ether | 60 | - | 0.07 5 | 0.13 | 0.4 |
| 8 | 0.2 mm; Paraffin oil | 60 | - | 1.4 | 2.5 | 3.7 |
| 9 | 0.2 mm, Rapeseed oil | 60 | - | 0.8 | 1.3 | 1.8 |
| 10 | 0.2 mm, Ethylene glycol | 20 | - | 0.8 | 1.6 | 2.2 |

### Bsp. 3

In diesem Beispiel werden die oberflächen-modifizierten Kalziumoxidpartikel hergestellt. Die nachstehende Tabelle zeigt die Process robustness unter verschiedenen Bedingungen.

| Nr | Mahlhilfsmittel | Zeit /min | Additiv | d10 / µm | d50 /µm | d90 / µm |
|---|---|---|---|---|---|---|
| 11 | 0.1 mm / 0.2 Rapeseed oil | 60 | - | 0.9 | 1.5 | 2.5 |
| 12 | 0.7 mm / Rapeseed oil | 60 | - | 0.9 | 1.5 | 2.2 |
| 13 | 0.7 mm / Rapeseed oil | 20 | Oleic acid, 2.5 mass-% | 1.2 | 2.1 | 3.7 |
| 14 | 0.7 mm / Rapeseed oil | 40 | Oleic acid, 2.5 mass-% | 1.2 | 2.0 | 3.8 |
| 15 | 0.7 mm / Rapeseed oil | 90 | Oleic acid, 2.5 mass-% | 0.9 | 1.6 | 2.3 |
| 16 | 0.3 mm / 0.4 Rapeseed oil | 60 | Lecithine, 2.5 mass-% | 0.9 | 1.5 | 2.2 |
| 17 | 0.7 mm / Rapeseed oil | 60 | Stearic acid, 2.5 mass-% | 3.0 | 5.6 | 6.9 |
| 18 | 0.7 mm / Rapeseed oil | 20 | Tetradecyl phosphonicacid, 0.25 mass-% | 2.4 | 4.5 | 6.6 |

Die Ergebnisse zeigen, dass die verschiedenen Additive für das erfindungsgemässe Verfahren geeignet sind. Ölsäure erscheint hier als besonders vorteilhaft wegen der engen Partikelgrössenverteilung.

### Bsp. 4 Anwendungen im Pflanzenschutz

Die nachfolgenden Beispiele zeigen, dass die erfindungsgemässen oberflächenmodifizierten Kalziumoxidpartikel eine breite fungizide und bakterizide Wirkung entfalten und daher im Pflanzenschutz vorteilhaft eingesetzt werden können. Die verwendeten Kalziumoxidpartikel wurden nach Methode (II), wie vorstehend dargestellt, erhalten und haben einen mittleren Partikeldurchmesser von 1 - 100 Mikrometer, bevorzugt 1 - 20 Mikrometer.

### 4.1: Feuerbrand

Fein gepulvertes CaO wurde mit Stearinsäure (5 Gew-% Stearinsäure bezogen auf CaO) beschichtet und als Staub auf Apfelblüten gegen Feuerbrand getestet. Die standardisierten Tests wurden bei einem dafür zertifizierten Institut (bioferm Research GmbH, DE) durchgeführt.

Beschichtung von CaO mit Stearinsäure: 20 g CaO Pulver wurde mit 1 g Stearinsäure als Lösung in Ethanol (20 ml) angefeuchtet, zu einer homogenen Masse vermengt. Das Ethanolfeuchte Pulver wurde am Rotationsverdampfer unter Wasser und Luftausschluss getrocknet, und ergab ein weisses Pulver als beschichtetes CaO.

Apfelblüten der Sorte Gala wurden mit dem Blütenstiel in einer 10%igen Saccharoselösung gestellt und in einer feuchten Kammer inkubiert.

Inokulation/Pathogen: Erwinia amylovora Stamm 385 (Ea385) wurde als Bakteriensuspension aus frischen Kulturen mit 10 Mio Zellen pro Milliliter auf die Blüten aufgesprüht. Infizierte Blüten wurden 1 Stunde nach Inokulation mit dem beschichteten CaO als Staub (Versuchsreihe) behandelt oder der Standard-Behandlung (Streptomycinsulfat 0.06% als Sprühlösung; Kontrolle und Benchmark) unterzogen. Auswertung: Die Blüten wurden nach 6 Tagen untersucht, und auf die Pathogen-typischen Merkmale einer Infektion untersucht (Schleimtropfen). 39% der Blüten ohne Behandlung waren befallen von Feuerbrand. Ausgewertet wurde die

Reduktion des Befalls durch die entsprechende Behandlung, als Wirkungsgrad der Behandlung

| | Wirkungsgrad der Behandlung [%] | Standardabweichung [%] |
|---|---|---|
| Streptomycinsulfat 0.06% | 68 | 14 |
| CaO Staub mit 5% Stearinsäure beschichtet | **82** | **7** |

### 4.2: Apfelschorf

Modifiziertes CaO wurde zur Behandlung von Apfelschorf (Venturia inaequalis) an einem dafür zugelassenen Institut (FiBL, CH) getestet.

### Beschichtetes CaO:

Beschichtung mit Stearinsäure: gemäss Beispiel 4.1.

Beschichtung mit Polymilchsäure: 20 g CaO Pulver wurde mit 1 g Polymilchsäure als Lösung in Chloroform (20 ml) angefeuchtet, zu einer homogenen Masse vermengt. Das feuchte Pulver wurde am Rotationsverdampfer unter Wasser und Luftausschluss getrocknet, und ergab ein weisses Pulver als beschichtetes CaO.

Pathogen: Venturia inaequalis.

Pflanzen: Apfelpflanzen der Sorte Jonagold wurden in Töpfen kultiviert und wuchsen bis zu einem 5-Blatt Stadium.

Inokulation: Die trockenen Pflanzen wurden mit Staub oder dem Standard-Mittel behandelt. Drei Stunden später wurden die Pflanzen an allen Blättern mit einer Suspension von 100 Tausend Keimen pro Milliliter besprüht.

Auswertung: Infizierte Pflanzen wurden als Prozent befallene Blätter (sichtbares Auftauchen der Krankheitssymptome) ausgewertet. Als Kontrollen für erfolgreiche Behandlungen wurde das marktübliche "Kocide Opti (ein Kupferpräparat)" entweder als 0.1 % oder 0.01 % ige Lösung verwendet. Der Schweregrad des Befalls (severity) wird als Anteil (%) der befallenen, toten Blätter angegeben.

| | Infizierte Blätter [%] | Standardabweichung [%] | Schweregrad [%] | Standardabweichung [%] |
|---|---|---|---|---|
| Keine Behandlung nach Inokulation | 55 | 11 | 28 | 8 |
| Standardbehandlung Kocide Opti 0.1 % | 17 | 11 | 1.6 | 2.3 |
| Standardbehandlung Kocide Opti 0.01 % | 43 | 8 | 12 | 4.6 |
| Vergleich 1 unbehandeltes CaO Pulver | 46 | 7 | 19 | 2.6 |
| Vergleich 2 Polymilchsäure-CaO | 52 | 7 | 26 | 3 |
| Erfindungsgemäss Stearinsäure-CaO | **26** | **6** | **8.7** | **5** |

### 4.3: Falscher Mehltau

Modifiziertes CaO wurde zur Behandlung von falschem Mehltau (Plasmopara viticola) an einem dafür zugelassenen Institut (FiBL, CH) getestet.

### Beschichtetes CaO:

Beschichtung mit Stearinsäure: gemäss Beispiel 4.1.

Beschichtung mit Polymilchsäure: gemäss Beispiel 4.2.

Pflanzen: Reben der Sorte Chasselas wurden in Töpfen kultiviert und wuchsen bis zu einem 4-Blatt Stadium.

Pathogen: Plasmopara viticola.

Inokulation: Die Pflanzen wurden feucht mit Staub oder dem Standard-Mittel behandelt. Dies simuliert eine vorbeugende Behandlung der Kulturen im Falle von den bei Mehltauverbreitung gefürchteten, feuchten Witterungsbedingungen dar. 3 Stunden später wurden Pflanzen an allen Blättern mit einer Suspension von 50 Tausend Sporen pro Milliliter besprüht.

Auswertung: Infizierte Pflanzen wurden als Prozent befallene Blätter (sichtbares Auftauchen der Krankheitssymptome) ausgewertet. Als Kontrollen für erfolgreiche Behandlungen wurde ein marktübliches Kupferpräparat (Kocide Opti) entweder als 0.1 % oder 0.01 % ige Lösung verwendet. Der Schweregrad des Befalls (severity) wurde als Anteil (in Prozent) der befallenen, toten Blätter erfasst und ist in nachstehender Tabelle angegeben.

| | Infizierte Blätter [%] | Standardabweichung [%] | Infizierte, tote Blätter (Schweregrad) | Standardabweichung [%] |
|---|---|---|---|---|
| Keine Behandlung nach Inokulation | 93 | 12 | 72 | 15 |
| Standardbehandlung Kocide Opti 0.1 % | 7.5 | 12 | 0.3 | 0.5 |
| Standardbehandlung Kocide Opti 0.01 % | 82.5 | 15 | 26 | 10 |
| Vergleich 1 Unbehandeltes CaO Pulver | 72 | 17 | 41 | 11 |
| Vergleich 2 Polymilchsäure-CaO | 92 | 13 | 67 | 8 |
| Erfindungsgemäss Stearinsäure-CaO | **43** | **40** | **16** | **18** |

In einem zweiten Teil der Versuche wurden die Pflanzen mit den gleichen CaO - Materialien direkt nach der Inokulation mit dem Mehltau - Erregern behandelt. Dies stellt die Feldbedingungen bei einer Behandlung von Kulturen während einer Verbreitung des Erregers nach.

| | Infizierte Blätter [%] | Standardabweichung [%] | Infizierte, tote Blätter (Schweregrad) | Standardabweichung [%] |
|---|---|---|---|---|
| Vergleich 1, post Unbehandeltes CaO Pulver | 96 | 9 | 45 | 17 |
| Vergleich 2, post Polymilchsäure-CaO | 93 | 10 | 52 | 9 |
| Stearinsäure-CaO, post | 57 | 27 | 7.3 | 5.9 |

## Patentansprüche

1. Oberflächenmodifizierte Kalziumoxidpartikel, **dadurch gekennzeichnet dass** diese
▪ mit einer mittleren Partikelgrösse von 1 bis 10 Mikrometer
▪ einem Gehalt an Ölsäure von 0.5 bis 5 %
▪ einem Gehalt an Kalziumoxid von 95 bis 99 % vorliegen; oder
▪ mit einer mittleren Partikelgrösse von 1 bis 10 Mikrometer
▪ einem Gehalt an Dodecyl-Phosphonsäure von 0.25 bis 2 %
▪ einem Gehalt an Kalziumoxid von 96 bis 99 % vorliegen; oder
▪ mit einer mittleren Partikelgrösse von 1 bis 10 Mikrometer
▪ einem Gehalt an Stearinsäure von 0.5 bis 5 %
▪ einem Gehalt an Kalziumoxid von 95 bis 99 % vorliegen; oder
▪ mit einer mittleren Partikelgrösse von 0.5 bis 2 Mikrometer,
▪ einem Gehalt an Ölsäure von 0.2 bis 5 %,
▪ einem Gehalt an Kalziumoxid in den Partikeln von 90 bis 99 % bildet
▪ als 5-20 m-% Dispersion in Rapsöl vorliegen.

2. Verwendung von oberflächenmodifizierten Kalziumoxidpartikeln nach Anspruch 1,
▪ als Pflanzenbehandlungsmittel;
▪ als Füllstoff in Kunststoffen;
▪ als Füllstoff und ggf. als Pigment in Lacken oder Farben;
▪ als Lebensmitteladditiv;
▪ als Additiv in Kosmetika;
▪ als Hilfsstoff in pharmazeutischen Produkten;
▪ als Additiv in Papiererzeugnissen;
▪ als Haftvermittler in Leim- und Polymerformulierungen;
▪ als Hilfsstoff in Zement- oder Betonmischungen.

3. Pflanzenbehandlungsmittel aus der Gruppe der Fungizide und Bakterizide enthaltend Kalziumoxidpartikel gemäss Anspruch 1 in einer Menge 1-20 m-%.

4. Kunststoff aus der Gruppe der Polyolefine, Polyether, Polysulfone, Polyamide, und Polyester, welche ggf. teilweise oder vollständig halogeniert sind, enthaltend Kalziumoxidpartikel gemäss Anspruch 1 in einer Menge von 0.2-40 m-%.

5. Lacke oder Farben aus der Gruppe Polyacrylate, Polyester und Polyurethane enthaltend Kalziumoxidpartikel gemäss Anspruch 1 in einer Menge von 0.2-40 m-%.

6. Lebensmittel aus der Gruppe der Back- und Süsswaren, der Fleisch- und Fischerzeugnisse und der alkoholischen Getränke, enthaltend Kalziumoxidpartikel gemäss Anspruch 1 in einer Menge von 0.05-2 m-%.

7. Kosmetischer Artikel aus der Gruppe der Hautpflegeprodukte und Zahnpflegeprodukte enthaltend Kalziumoxidpartikel gemäss Anspruch 1 in einer Menge von 0.5-20 m-%.

8. Pharmazeutische Formulierung aus der Gruppe der festen Formulierungen enthaltend Kalziumoxidpartikel gemäss Anspruch 1 in einer Menge von 0.05 bis 10 m-%.

9. Papiererzeugnis enthaltend Kalziumoxidpartikel gemäss Anspruch 1 einer oberen Schicht; insbesondere Papier für Tintenstrahldrucker enthaltend Kalziumoxidpartikel gemäss Anspruch 1 in einer oberen, die Tinte aufnehmenden, Schicht.

10. Leim- und Polymerformulierungen enthaltend Kalziumoxidpartikel gemäss Anspruch 1 in einer Menge 0.5 - 40 m-%.

11. Verwendung von oberflächenmodifizierten Kalziumoxidpartikeln gemäss Anspruch 1 oder 15 als Fungizid und / oder Bakterizid, insbesondere im Obst-, Gemüse- und Weinbau.

12. Verfahren zur Herstellung von oberflächenmodifizierten Kalziumoxidpartikeln nach Anspruch 1, insbesondere ein kontinuierliches Verfahren, umfassend die Schritte
- Methode I -
(a) Bereitstellen von rohem Kalziumoxid, Additiv, Mahlhilfsmittel,
(b) Bereitstellen und Inertisieren einer gegen die Umgebung abgedichteten Mahlvorrichtung (M),
(c) Mahlen des rohen Kalziumoxids in Gegenwart von Mahlhilfsmittel und Additiv in dieser Mahlvorrichtung; oder
- Methode II -
(a) Bereitstellen von rohem Kalziumoxid, Additiv, Mahlhilfsmittel,
(b) Bereitstellen und Inertisieren von gegen die Umgebung abgedichteten Mahlvorrichtung (M) und Sprühvorrichtung (S),
(c) Mahlen des rohen Kalziumoxids in Gegenwart von Mahlhilfsmittel in besagter Mahlvorrichtung (M),
(d) Kontaktieren des gemahlenen Kalziumoxids mit Additiv in besagter Sprühvorrichtung (S);
wobei das Mahlhilfsmittel (i) ein inertes Gas mit einem Wassergehalt <0.1g/m³ ist, oder (ii) eine nichtwässrige Flüssigkeit mit einem Wassergehalt <0.1g/l ist; und wobei das Additiv eine Flüssigkeit ist welche Verbindungen enthält die ausgewählt sind aus der Gruppe umfassend Ölsäure, Stearinsäure und Dodecyl-Phosphonsäure; und
wobei die Methode I und II gegebenenfalls zusätzlich umfassen:
(f) Abtrennen des oberflächenmodifizierten Kalziumoxids in einer Trennvorrichtung (T) und gegebenenfalls
(g) Rezyklieren des Mahlhilfsmittels und / oder überschüssigem Additivs, ggf. unter Kühlung in einem Wärmetauscher (W).

13. Verfahren nach Anspruch 12,
wobei in Schritt (a) das rohe Kalziumoxid gebrannter Kalk ist, welcher aus natürlichem Kalkstein gewonnen ist; und / oder
wobei in Schritt (b) das Inertisieren (Trocknen) erfolgt, indem
▪ die Mahlvorrichtung und ggf. Sprühvorrichtung erhitzt werden (bevorzugt 120°C, 1h) und / oder
▪ Schritt (c) durchgeführt wird, bis ein Produkt gleichbleibender Qualität erzeugt wird; und / oder
wobei in Schritt (c) das Mahlen entweder in einer Gasstrahlmühle erfolgt, oder in einer Nassmühle erfolgt und wobei die Ausgangsmaterialien Kalziumoxid, Additiv und Mahlhilfsmittel gleichzeitig der Mühle zugeführt werden; oder
wobei in Schritt (c) das Mahlen entweder in einer Gasstrahlmühle erfolgt, oder in einer Nassmühle erfolgt und wobei die Ausgangsmaterialien Kalziumoxid, und Mahlhilfsmittel gleichzeitig der Mühle zugeführt werden und wobei in Schritt (d) das Additiv zugeführt wird.

14. Verfahren nach Anspruch 12, in welchem
▪ der Gehalt an Additiv in den oberflächenmodifizierten Kalziumoxidpartikeln < 20 m-% ist, und /oder
▪ die oberflächenmodifizierten Kalziumoxidpartikel einen mittleren Durchmesser von < 10 µm aufweisen, und / oder
▪ das rohe Kalziumoxid in Partikeln mit einer maximalen Grösse von 200 µm vorliegt.

## Claims

1. Surface-modified calcium oxide particles, **characterized in that** these are present:
• with a mean particle size of 1 to 10 micrometers;
• a content of oleic acid of 0.5 to 5%;
• a content of calcium oxide of 95 to 99%,
or
• with a mean particle size of 1 to 10 micrometers;
• a content of dodecylphosphonic acid of 0.25 to 2%;
• a content of calcium oxide of 96 to 99%,
or
• with a mean particle size of 1 to 10 micrometers;
• a content of stearic acid of 0.5 to 5%;
• a content of calcium oxide of 95 to 99%,
or
• with a mean particle size of 0.5 to 2 micrometers;
• a content of oleic acid of 0.2 to 5%;
• a content of calcium oxide in the particles of 90 to 99% forms;
• as a 5-20% by mass dispersion in rapeseed oil.

2. Use of surface-modified calcium oxide particles according to claim 1
• as plant treatment means;
• as filler in plastics;
• as filler and, optionally, pigment in varnishes or paints;
• as a food additive;
• as an additive in cosmetics;
• as an auxiliary agent in pharmaceutical products;
• as an additive in paper products;
• as adhesion promoters in adhesive and polymer formulations;
• as an auxiliary agent in cement or concrete mixtures.

3. Plant treatment means from the group of fungicides and bactericides containing calcium oxide particles according to claim 1 in a quantity of 1-20% by mass.

4. Plastic from the group of polyolefins, polyethers, polysulfones, polyamides, and polyesters, which, optionally, are partially or fully halogenated, containing calcium oxide particles according to claim 1 in a quantity of 0.2-40% by mass.

5. Varnishes or paints from the group of polyacrylates, polyesters, and polyurethanes containing calcium oxide particles according to claim 1 in a quantity of 0.2-40% by mass.

6. Foodstuffs from the group of baked goods and confectionery, meat and fish products, and alcoholic beverages, containing calcium oxide particles according to claim 1 in a quantity of 0.05-2% by mass.

7. Cosmetic articles from the group of skin care products and dental care products containing calcium oxide particles according to claim 1 in a quantity of 0.5-20% by mass.

8. Pharmaceutical formulations from the group of solid formulations containing calcium oxide particles according to claim 1 in a quantity of 0.05 to 10% by mass.

9. Paper product containing calcium oxide particles according to claim 1 in an upper layer; in particular paper for inkjet printers containing calcium oxide particles according to claim 1 in an upper layer, which receives the ink.

10. Adhesive and polymer formulations containing calcium oxide particles according to claim 1 in a quantity of 0.5-40% by mass.

11. Use of surface-modified calcium oxide particles according to claim 1 or 15 as a fungicide and/or bactericide, in particular in fruit and vegetable farming and viniculture.

12. Method for producing surface-modified calcium oxide particles according to claim 1, particularly a continuous method, comprising the steps of:
(Method I)
(a) providing raw calcium oxide, an additive, and a milling aid;
(b) providing and inertizing a milling device (M), which is sealed with respect to the surroundings,
(c) milling the raw calcium oxide in the presence of the milling aid and the additive in this milling device; or
(Method II)
(a) providing raw calcium oxide, an additive, and a milling aid;
(b) providing and inertizing a milling device (M), which is sealed with respect to the surroundings, and a spraying device (S),
(c) milling the raw calcium oxide in the presence of the milling aid in said milling device (M),
(d) contacting the milled calcium oxide with the additive in said spraying device (S);
wherein the milling aid (i) is an inert gas having a water content of < 0.1g/m³, or (ii) is a non-aqueous liquid having a water content of < 0.1g/l; and
wherein the additive is a liquid, which contains compounds selected from the group of fatty acid, stearic acid and dodecylphosphonic acid; and
wherein the method I and II optionally additionally comprise:
(f) Separating the surface-modified calcium oxide in a separation device (T) and optionally
(g) Recycling the milling aid and/or excessive additive, optionally by cooling in a heat exchanger (W).

13. Method according to claim 12,
wherein in step (a) the raw calcium oxide is burnt lime, which is obtained from natural limestone; and/or
wherein inertizing (drying) is carried out in step (b), such that:
• the milling device and, optionally, the spraying device are heated (preferably 120°C, 1h) and/or
• step (c) is carried out until a product having a consistent quality is produced; and/or
wherein in step (c) the milling takes place either in a gas jet mill or in a wet mill, and wherein the starting materials calcium oxide, additive, and milling aid are fed to the mill simultaneously; or
wherein in step (c), the milling takes place either in a gas jet mill or in a wet mill, and wherein the starting materials calcium oxide and milling aid are fed to the mill simultaneously and wherein in step (d) the additive is supplied.

14. Method according to claim 12, wherein:
• the content of additive in the surface-modified calcium oxide particles is < 20% by mass, and/or
• the surface-modified calcium oxide particles have a mean diameter of < 10 µm, and/or
• the raw calcium oxide is present in particles having a maximum size of 200 micrometers.

## Revendications

1. Particules d'oxyde de calcium à surface modifiée, **caractérisés en ce que** ils sont présents:
• avec une moyenne taille de particules comprise entre 1 et 10 micromètres;
• un contenu d'acide oléique compris entre 0.5 et 5%;
• un contenu d'oxyde de calcium compris entre 95 et 99%, ou
• avec une moyenne taille de particules comprise entre 1 et 10 micromètres;
• un contenu d'acide dodécylphosphonique compris entre 0.25 et 2%;
• un contenu d'oxyde de calcium compris entre 96 et 99%, ou
• avec une moyenne taille de particules comprise entre 1 et 10 micromètres;
• un contenu d'acide stéarique compris entre 0.5 et 5%;
• un contenu d'oxyde de calcium compris entre 95 et 99%, ou
• avec une moyenne taille de particules comprise entre 0.5 et 2 micromètres;
• un contenu d'acide oléique compris entre 0.2 et 5%;
• un contenu d'oxyde de calcium formant dans les particules compris entre 90 et 99%;
• comme une dispersion de 5-20% par poids dans d'huile de colza.

2. Utilisation des particules d'oxyde de calcium à surface modifiée selon la revendication 1
• comme moyen de traitement des plantes;
• comme produit de remplissage dans les plastiques;
• comme produit de remplissage et, optionnellement, pigment dans des vernis ou laques;
• comme additif alimentaire;
• comme additif dans des produits cosmetiques;
• comme un agent auxiliaire dans des produits pharmaceutiques;
• comme un additif dans des produits de papier;
• comme promoteurs d'adhérence dans des formulations d'adhésif et de polymères;
• comme un agent auxiliaire dans des mélanges de cément ou de béton.

3. Moyen de traitement de plantes du groupe de fongicides et bactéricides contenant des particules d'oxyde de calcium selon la revendication 1 dans une quantité de 1-20% par poids.

4. Plastique du groupe de polyoléfines, polyéthers, polysulphones, polyamides, et polyesters, qui sont optionnellement partiellement ou entièrement halogénés, contenant des particules d'oxyde de calcium selon la revendication 1 dans une quantité de 0.2-40% par poids.

5. Vernis ou laque du groupe de polyacrylates, polyesters, et polyuréthanes contenant des particules d'oxyde de calcium selon la revendication 1 dans une quantité de 0.2-40% par poids.

6. Produits alimentaires du groupe de produits de boulangerie et confiserie, de produits de viande et poisson, et des boissons alcooliques, contenant des particules d'oxyde de calcium selon la revendication 1 dans une quantité de 0.05-2% par poids.

7. Produits cosmétiques du groupe de produits de soin de peau et de produits de soin dentaire contenant des particules d'oxyde de calcium selon la revendication 1 dans une quantité de 0.5-20% par poids.

8. Formulations pharmaceutiques du groupe de formulations solides contenant des particules d'oxyde de calcium selon la revendication 1 dans une quantité de 0.05 à 10% par poids.

9. Produit de papier des particules d'oxyde de calcium selon la revendication 1 dans une couche supérieure; particulièrement du papier pour des imprimantes à jet d'encre contenant des particules d'oxyde de calcium selon la revendication 1 dans une couche supérieure qui reçoit l'encre.

10. Formulations d'adhésif et de polymères contenant des particules d'oxyde de calcium selon la revendication 1 dans une quantité de 0.5-40% par poids.

11. Utilisation des particules d'oxyde de calcium à surface modifiée selon la revendication 1 ou 15 comme un fongicide et/ou bactéricide, particulièrement dans l'agriculture de fruits et légumes et dans la viniculture.

12. Procédé de production des particules d'oxyde de calcium à surface modifiée selon la revendication 1, particulièrement un procédé continu, comprenant les étapes:
(Procédé I)
(a) de fournir d'oxyde de calcium brut, un additif, et un adjuvant de broyage;
(b) de fournir et inertiser un dispositive de broyage (M) qui est scellé par rapport à l'environnement,
(c) de broyer l'oxyde de calcium brut en présence de l'adjuvant de broyage et de l'additif dans ce dispositif de broyage; ou
(Procédé II)
(a) de fournir d'oxyde de calcium brut, un additif, et un adjuvant de broyage;
(b) de fournir et inertiser un dispositive de broyage (M) qui est scellé par rapport à l'environnement, et un dispositif de pulvérisation (S),
(c) de broyer l'oxyde de calcium brut en présence de l'adjuvant de broyage dans ce dispositif de broyage (M),
(d) de contacter l'oxyde de calcium broyé avec l'additif dans ledit dispositif de pulvérisation (S);
l'adjuvant de broyage (i) étant un gaz inerte ayant un contenu d'eau de < 0.1g/m³, ou (ii) étant un liquide non-aqueux ayant un contenu d'eau de < 0.1g/l; et
l'additif étant un liquide qui contient des composés sélectionnés du the group d'acides gras, d'acides stéariques et d'acides dodécylphosphoniques; et
le procédé I et II optionnellement comprenant en outre:
(f) séparer l'oxyde de calcium à surface modifiée dans un dispositif de séparation (T) et optionnellement
(g) recycler l'adjuvant de broyage et/ou de l'additif excessif, optionnellement par refroidissement dans un échangeur de chaleur (W).

13. Procédé selon la revendication 12,
à l'étape (a) l'oxyde de calcium brut étant de la chaux brulée obtenue du calcaire naturel; et/ou
l'inertisation (séchage) étant effectuée à l'étape (b), de sorte que:
• l'adjuvant de broyage et, optionnellement, le dispositif de pulvérisation sont chauffés (préférablement 120°C, 1h) et/ou
• l'étape (c) est effectuée jusqu'à un produit ayant une qualité consistante est produit; et/ou
à l'étape (c) le broyage se déroulant soit dans un broyeur à jet de gaz ou dans un broyeur humide, et les matériaux initiaux oxyde de calcium, additif, et adjuvant de broyage étant alimentés dans le broyeur simultanément; ou
à l'étape (c), le broyage se déroulant soit dans un broyeur à jet de gaz ou dans un broyeur humide, et les matériaux initiaux oxyde de calcium et adjuvant de broyage étant alimentés dans le broyeur simultanément et à l'étape (d) l'additif étant alimenté.

14. Procédé selon la revendication 12:
• le contenu d'additif dans les particules d'oxyde de calcium à surface modifiée étant < 20% par poids, et/ou
• les particules d'oxyde de calcium à surface modifiée ayant un diamètre moyen de < 10 µm, et/ou
• l'oxyde de calcium brut étant présent dans des particules ayant une taille maximale de 200 micromètres.
